# EUROPEAN PATENT SPECIFICATION

(11) **EP 1 743 029 B1**
(45) Date of publication and mention of the grant of the patent: **29.06.2011**
(21) Application number: 05741716.4
(22) Date of filing: 05.05.2005
(51) Int. Cl.: C12N 15/867

(54) **CELL PREPARATION**
ZELLPRÄPARATION
PREPARATION DE CELLULES

(30) Priority: 06.05.2004 GB 0410130
(43) Date of publication of application: 17.01.2007
(73) Proprietor: Molmed SpA, 20132 Milan (IT)
(72) Inventor: BENATI, Claudia, I-20132 Milan (IT); SCIARRETTA BIROLO, Roberto, I-20132 Milan (IT); LA SETA CATAMANCIO, Simona, I-20132 Milan (IT); RADRIZZANI, Marina, I-20132 Milan (IT); SENDRESEN, Cecilia, I-20132 Milan (IT); TOMA, Salvatore, I-20132 Milan (IT)
(74) Representative: Mallalieu, Catherine Louise
(86) International application number: PCT/IB2005/001565
(87) International publication number: WO 2005/108589

(56) References cited:
- ROBINET E, ET AL.: "PREPARATION DE PRODUITS DE THERAPIE CELLULAIRE: APPORT DES SYSTEMES CLOS" TRANSFUS. CLIN. BIOL., vol. 6, 1999, pages 409-417, XP009052792
- ROBINET E, ET AL.: "A CLOSED CULTURE SYSTEM FOR THE EX VIVO TRANSDUCTION AND EXPANSION OF HUMAN T LYMPHOCYTES" JOURNAL OF HEMATOTHERAPY, vol. 7, 1998, pages 205-215, XP009052788
- SLAPER-CORTENBACH: "HIGHLY ENRICHED, LNGF-R TRANSDUCED T CELL ISOLATION PROCEDURE IN A CLOSED SYSTEM USING ANTI-BIOTIN MICROBEADS AND THE NEW CLINIMACSPLUS 5.1. ENRICHMENT PROCEDURE" BLOOD, vol. 98, no. 11, 2001, page 411B, XP009052858
- FREIMARK B D, ET AL.: "COMPARISON OF RETROVIRAL TRANSDUCTION EFFICIENCIES OF HUMAN CD34 CELLS IN CLOSED CULTURE SYSTEMS" BLOOD, vol. 94, no. 10, 1999, page 410B, XP009052856
- THORNTON J, ET AL.: "INCREASED RETROVIRAL GENE TRANSFER INTO HUMAN CD34+ CELLS AND T-CELLS BY INCORPORATION OF A LOW-SPEED CENTRIFUGATION STEP IN A CLOSED-SYSTEM ENVIRONMENT" BLOOD, vol. 96, no. 11, 2000, page 384B, XP009052857

## Description

### Field of the Invention

The present invention relates to methods for transduction and selection of genetically modified cells in a closed system.

### Background to the Invention

Currently an open system is usually used for the transduction and selection of genetically modified cells. However, such systems can be time consuming and can be associated with significant contamination risks. This is particularly the case when the cells are to be used in a clinical setting and when high doses are required. An example of such a requirement is the production of donor T-cells for use in allogeneic bone marrow transplantation.

Allogeneic bone marrow transplantation is the treatment of choice for many hematologic malignancies (Thomas 1983, J Clin Oncol 1:517; O'Reilly 1993, Curr Op In Hematol 221).

In the context of allogeneic bone marrow transplantation, the immunological role of donor T lymphocytes in the often complete eradication of the tumor is universally recognized. The antitumor effect derived from the donor T lymphocytes (Graft-versus-Leukemia, GvL) renders this therapeutic method superior to conventional chemotherapy and autologous transplantation.

Moreover, the infusion of donor T lymphocytes is also able to mediate the reconstitution of immune responses against viruses and fungi, which is demonstrated by the lower incidence and severity of such infections in the context of the non-manipulated transplantation versus those which have been depleted for T lymphocyte (Papadopoulos 1994, N Engl J Med 330:1185; Rooney 1995, The Lancet 345:9; Heslop 1996, Nature Med 2:551; Rooney 1998, Blood 92:1549; Riddel 1992, Science 257; Walter 1995, N Engl J Med 333:1038).

In the face of a clear clinical benefit in the induction of an antitumor effect, the infusion of donor T lymphocytes is still complicated by an elevated risk for the development of graft versus host disease (GvHD). This is due to the aggression against the host tissues by the donor lymphocytes and is characterized by an elevated death and morbidity, especially in patients who receive the transplant from a related haploidentical donor (Kolb 1995, Blood 86:2041; Collins 1997, J Clin Oncol 15:433; Porter 2000, Blood 95:1214).

The incidence and severity of the disease is proportional to the number of infused lymphocytes, meaning that the larger the dose, the more severe the disease. Since the clinical benefit in terms of antitumoral and antiviral activity is proportional to the infusion dose, increasing the dose augments the antitumoral benefit but at the same time the risk of GvHD. A specific treatment for GvHD doesn't exist and those therapies in use, based on steroids and other immunosuppressants, are nonspecific and complicated by an elevated incidence of severe infections and disease relapse.

In order to obtain immunological reconstitution and reduce the incidence of infectious events and disease relapse while being able to selectively control emerging GvHD, a promising strategy is the use of donor lymphocytes that have been transduced with a retroviral vector containing suicide and marker genes. The suicide gene renders genetically modified cells sensitive to a drug that will later be used to selectively eliminate infused cells in case of emerging GvHD. The presence of a marker gene permits the following of survival, expansion and sites of migration of genetically modified cells.

For example, the vector SFCMM-3 may be used, which carries both the suicide gene HSV-tk and the marker gene ALNGFR (Verzeletti 98, Human Gene Therapy 9:2243). HSV-tk encodes the thymidine kinase enzyme of Herpes Simplex Virus I (HSV-tk) which once inserted into donor lymphocytes renders them selectively sensitive to ganciclovir. The drug ganciclovir, after administration to the patient, is phosphorylated by the thymidine kinase enzyme expressed by the genetically modified cells and then successively by cellular kinases. The active form of ganciclovir inhibits the synthesis of genomic DNA, thereby causing cell death (Smee 1983, Antimicrobials Agents and Chemotherapy 4:504). The HSV-tk suicide system has already been demonstrated to be efficient in diverse clinical studies (Bordignon 1995, Hum Gene Ther 2:813; Bonini 1997, Science 276:1719; Tiberghien 1997, Hum Gene Ther 8:615; Tiberghien 2001, Blood 97:63; Link 1998, Hum Gene Ther 9:115; Champlin 1999, Blood 94:1448).

The LNGFR gene encodes the low affinity receptor for nerve growth factor (NGF) which has been deleted in the intracellular portion such that it is no longer able to transmit signals (ALNGFR) (Mavilio 1994, Blood 83:1988). Utilizing monoclonal antibodies and magnetic beads, the presence of the ALNGFR protein allows for in vitro immunoselection of the genetically modified cells. Furthermore, the expression of the ALNGFR protein is used as a marker for genetically modified cells once infused into the patient to allow for documentation of the presence, expansion or reduction of such cells and for their characterization in terms of lymphocyte subtypes and state of activation.

The clinical studies indicated above (Bordignon 1995, Hum Gene Ther 2:813; Bonini 1997, Science 276:1719; Tiberghien 1997, Hum Gene Ther 8:615; Tiberghien 2001, Blood 97:63; Link 1998, Hum Gene Ther 9:115; Champlin 1999, Blood 94:1448) rely on different methods for production of genetically-modified lymphocytes (the final product). The methods include cell thawing, stimulation, transduction, selection, expansion and recovery of the final product for patient infusion. The safety of the final product, tested before patient infusion, is related to cell manipulation during each step of the process. Usually these methods are developed in open systems using flasks for cell culture and laminar flow boxes for cell manipulations such as transduction and selection. Currently an open system is used for production of transduced T cells for clinical protocols. However, such a system can be time consuming and can be associated with significant contamination risks.

There is thus a need for an efficient and safe method of transducing and selecting genetically modified cells.

### Summary of the Invention

The present invention overcomes the aforementioned problem by.providing a method for cell manipulation and culturing in a closed system. The transformation from an open system to a closed system allows for scale-up of the product and improvement of the safety of the product for clinical use.

In one aspect of the present invention there is provided a method for transduction of cells with a genetic construct and selection of genetically modified cells, the method comprising performing the transduction and selection in a closed system, wherein the cells are washed, concentrated, transduced and re-suspended using an automated fluid management device without any manual transfer of fluids, and wherein the method comprises immunomagenetic selection of the transduced cells.

In another aspect of the present invention there is provided a method of modifying cells comprising:
(i) optionally thawing said cells;
(ii) optionally stimulating said cells;
(iii) transducing said cells with a genetic construct;
(iv) selecting transduced cells;
(v) optionally expanding and harvesting said transduced cells;
(vi) wherein steps (i) to (v) are performed in a closed system, and wherein step (iv) comprises immunomagenetic selection of transduced cells; and
(vii) wherein the cells are washed, concentrated, transduced and re-suspended using an automated fluid management device without any manual transfer of fluids, and wherein the method comprises immunomagenetic selection of the transduced cells.

A closed system is an isolated system that prevents exposure of the cells to the environment outside of the system. The cells are only exposed to the immediate environment of the bags, tubing and machine components that make up the closed system.

The closed system of the present invention prevents contamination of the transduced cells. It achieves this by ensuring that the cells are sealed off from the environment external to the system, preventing contaminants from entering the system.

In one embodiment the genetic construct is a retroviral vector.

Preferably, the retroviral vector is a Murine leukemia virus (MLV) derived vector.

Preferably the retroviral vector encodes a cell surface marker, such as, for example, LNGFR, which can be recognized by specific antibody.

In one embodiment the cells are donor T-cells.

Preferably, the method comprises a transduction step using fibronectin or a variant thereof. Previous studies suggested that some chymotryptic fragments of extra cellular matrix molecule can increase transduction of human hematopoietic progenitor cells when they are used during infection (Moritz et al. (1994) J. Clin. Invest. 93, 1451; Moritz, et al.,. (1996) Blood 88, 855). Takara has developed a recombinant peptide of human fibronectin, CH-296 (RetroNectin^{®)}, which consists of three functional domains (Hanenberg et al., (1996) Nature Medicine 2, 876) and has been shown to enhance retrovirus-mediated gene transduction (Kimizuka et al.,. (1991) J. Biochem.~110, 284).When coated on the surface of Petri dishes or flasks or bags, RetroNectin^{®} significantly enhances retrovirus-mediated gene transduction into mammalian cells.

Preferably, the RetroNectin® transduction system is used in the present invention.

The method of the present invention comprises immunomagnetic selection. Immunomagnetic selection refers to the coupling of antibodies to paramagnetic particles enabling a separation of the antigenic structures by use of a magnet. For example, a genetically-modified subpopulation of transduced cells expressing the ΔLNGFR cell surface molecule may be incubated with a mouse IgG anti-NGF receptor antibody that binds to the ΔLNGFR positive cells. The cells may then be incubated with immunomagnetic beads coated with sheep anti-mouse IgG, and applied to a magnet in order to isolate ΔLNGFR positive cells. The isolated cells may be recovered by switching off the magnet.

The closed system may comprise a single device that performs all of the steps necessary for arriving at isolated transduced cells.

Alternatively, the closed system may comprise two or more devices. A first device may be used for the cell concentration, cell washing, transduction and medium changing steps and a second device may-perform the selection of the transduced cells. Transfer of cells between the devices may be achieved by using sealed bags and aseptic connections so as to ensure the overall process remains a closed process. Preferably, the cells are washed, concentrated, transduced and re-suspended by using an automated fluid management device for cell manipulation. The device may consist of a spinning membrane that ensures cell filtration against a counter-flow buffer circulation, and which is connected to different bags, in a completely closed system that allows for step-by-step user-definable programming. Cell washing and concentration may be performed in a sterile disposable set consisting of the spinning membrane connected to a filtered wash bag, a buffer bag and a waste bag. The desired washing procedure may be defined by the user. An example of such a device is the Cytomate™ Cell Processing System.

The final product of the previous process may be concentrated in a bag. The bag may then be aseptically connected to a second tubing set in order to start the selection step using paramagnetic microspheres (beads). An example of a device suitable for this step is the Tsolex™ 300 Magnetic Cell Separation System.

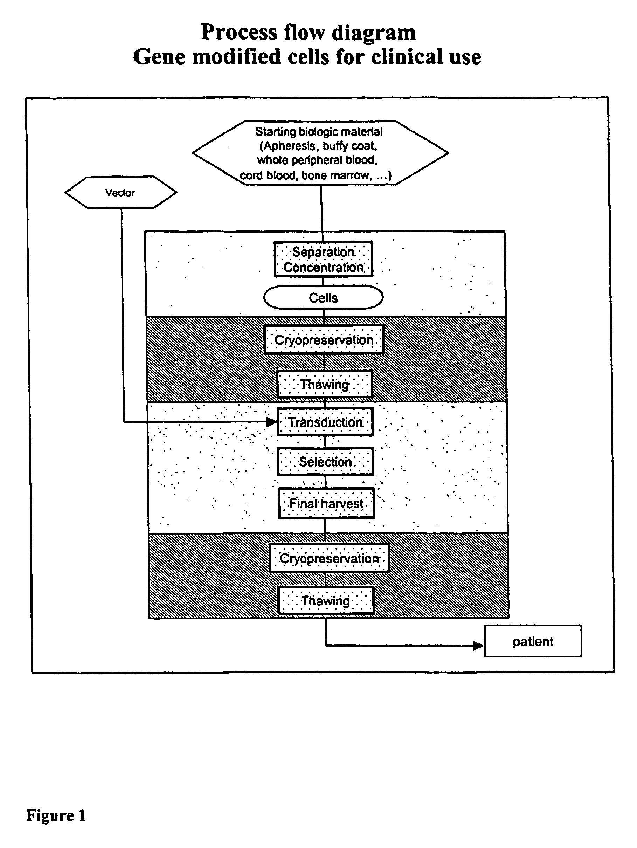

The process scheme shown in Figure 1 summarizes the steps that may be required for the preparation of the genetically-modified cells according to the present invention.

Depending on the clinical study, different biological starting materials can be manipulated. One or more steps may be omitted. In one embodiment, the steps in the darkened areas can be omitted.

The invention relates to methods for transduction and selection of genetically modified cells, preferably for clinical use, in a closed system.

Particularly the invention may include successive steps of thawing (required only in case frozen cells are used as starting material), stimulation (if required on the basis of cell and vector characteristics), transduction and selection (e.g., retroviral vectors are used), expansion and harvest of patient-specific cells for clinical use. The whole process can be conducted in a short time period, for example less than two weeks.

A closed system may be used for every step, including cell washing, media changing, incubation for transduction and selection and final harvesting for cell infusion into patient.

In one embodiment, the safety of the final product is assured by the closed system developed using disposable plastic materials and devices able to perform two different kinds of operations: 1) cell concentration/washing/medium changing; 2) magnetic selection.

In particular, the present invention may be successfully used for the production of genetically modified T cells to be used in the context of allogeneic bone marrow transplantation clinical protocols.

For example, the following devices may be used:
1) Cytomate™ Cell Processing System (trade mark of Nexell Therapeutic Inc.) (Baxter, code R4R9860), for cell manipulation in clinical grade closed disposable kits (Cell Washer Disposable Set, Baxter, code R4R9811);
2) Isolex™ 300 Magnetic Cell Separation System, for selection of the transduced cells.

Examples of CytoMate™ Cell Processing System used for washing of cells after thawing and examples of magnetic cell selector use for clinical scale isolation of cells are given in Calmels 2003, Bone Marrow Transplant 31:823 and Suen 2001, Cytotherapy 3:365 respectively.

The present invention groups the different steps of thawing, stimulation, transduction, selection, expansion of the cells and recovery of the final product for patient infusion in a single method, using a completely closed system that could be standardized for an easy manipulation of cells in clinical centers.

Advantages of the present invention include combination of different steps in a single method, the improvement of the final product safety and the easy manipulation of the cells.

Further preferred features and embodiments of the present invention will now be described by way of non-limiting example and with reference to the accompanying drawings in which:
Figure 1 shows a process scheme summarising the steps that may be required for the preparation of genetically modified cells.
Figure 2 shows the effect of the stimulation and transduction methods on cell expansion. Fold expansion was evaluated counting the cells at each indicated time point and dividing by the number of cell seeded at day 0.
Figure 3 shows the effect of the stimulation and transduction methods on transduction efficiency. % LNGFR positive cells at day 6 was evaluated by flow cytometry.
Figure 4 shows the effect of bead: cell ratio on cell expansion. Fold expansion was evaluated counting the cells at each indicated time point and dividing viable cell number by the number of cell seeded at day 0.
Figure 5 shows the effect of bead: cell ratio on transduction efficiency. % LNGFR positive cells at day 6 was evaluated by flow cytometry.

### Example 1

### Washing out DMSO from donor lymphocytes after thawing.

The present invention provides a method for cell manipulation and culturing in a completely closed system with the use of a device for cell manipulation in clinical grade closed disposable kits.

In this example the CytoMate™ was used as an automated fluid management device for cell manipulation. It consists of a spinning membrane that ensures cell filtration against a counter-flow buffer circulation and is connected to different bags, in a completely closed system that allows for a step-by-step user-definable programming.

Cell washing and concentration are performed in a sterile disposable set consisting of the spinning membrane connected to a filtered wash bag, a buffer bag and a waste bag. This system allows for cell processing in a GMP environment. The washing procedure is defined by the user, the bag containing the sample (to be processed) is connected to the sterile set and the fluid transfer, between the different bags, defined by the procedure is monitored by four weight scales and probes.

A simple menu-driven interface allows for programming of up to 100 custom procedure.

The washing efficiency is defined setting the "residual fold reduction" parameter in the washing program.

The starting material for the production of genetically-modified cells defined above may consist of fresh or frozen cells (from apheresis, buffy-coat, whole peripheral blood, cord blood, bone marrow), when frozen cells are used, they are thawed and the DMSO, a cryoprotective component (10%) of the freezing medium, is washed out after thawing in order to stimulate the cells for cell culturing.

After connection of the sample bag (or bags) to the cell source line of the disposable set (Baxter code R4R9811), cells are diluted twice with cold thawing buffer (X-Vivo 15, plus glutamine 1% and IL-2 600 IU) firstly with 10 ml at a rate of 5 ml/min and then with 20 ml at a rate of 10ml/min. During these operations the thawed bag (or bags) is layered on an ice stratum or on an ice pack and is gently shaken in order to resuspend the cells and to avoid cell sedimentation.

Then the cells are washed and concentrated with the use of the spinning membrane and transferred into a collection bag; the source bag, the washing bag and tubing are rinsed with 130 ml of buffer in order to minimize cell loss. Briefly, the cell suspension passes through the spinning membrane where the cells are concentrated outside of the membrane and passes out the top port while the supernatant containing cell debris and DMSO is drawn through the membrane out the bottom port.

The efficiency and length of the washing step depend on the selected parameter, the "residual fold reduction", that defines the degree of washing, the extent to which the original fluid is removed during the wash procedure. This parameter value is selectable from 1 to 1000. The value of 100 was chosen on the basis of Nexell data, CytoMate™ Custom Programming Guidelines; this value will result in approximately 2-log reduction of the source solution. The final volume, usually between 100 and 250 ml, depends on two selectable parameters: the initial cell number and the maximum final weight of the wash bag, the value of 250 was chosen as default setting on the basis of Nexell data, CytoMate™ Custom Programming Guidelines.

At the end of the washing procedure a sample of cells can be collected and evaluated for cell viability and number of cells; then the cells suspension can be diluted, according to the cell number, with the washing buffer that is the medium used for cell culturing; the cells are prepared for stimulation at a concentration of 1x10⁶/ml in culture medium.

The time length of the washing procedure including final cell dilution is approximately 1h.

Two different experiments were conducted using the washing procedure described above, with a single wash step. In these experiments different parameters were evaluated: starting and final volume, in order to evaluate washing feasibility of the starting samples with small volume of cell suspension; starting and final number of cells, in order to evaluate cell loss during thawing and washing steps; the length of the total procedure, in order to understand if the procedure is less time-consuming compared to an open system; the volume of the washing buffer used, in order to evaluate the reagents consumption.

In the experiments described below, the starting material is a bag of frozen PBL from healthy donor buffy-coat.

The volume of the starting material is 15ml but a large range of cell volumes can be treated in one or more bags.

The number of cells in the bag are approximately 1x10⁹ total, but the range of cell number to be treated can be between 0.1x10⁹ to 100x10⁹ contained in one or more bags.

The value of 2-log reduction ( the "residual fold reduction" value 100, set by the user in the washing program) of the source solution (containing DMSO and cellular debris) was chosen according to the literature data on DMSO washing out using CytoMate™ (Calmels B. et al., Bone Marrow Transplant. 2003 May;31 (9):823-8), but the value 50 is already enough to eliminate more than 96% of DMSO.

Table 1 shows the results obtained in three independent experiments using cells from buffy-coat of different donors.

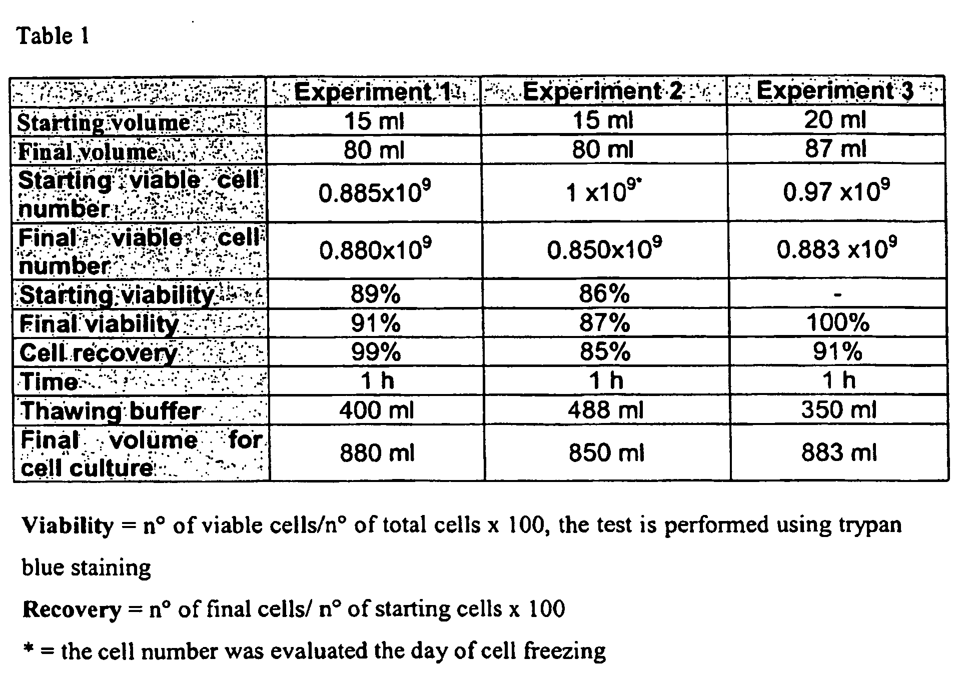

An alternative method for reducing the source solution would be to set a program with a secondary wash. Every program is step-by-step user definable.

The procedure follows the same principles described above but with the addition of a secondary wash step. In this case, the length of the procedure is extended to 1 hand 20' and the volume of thawing buffer increases to 1100 ml.

Three experiments where performed using a procedure with the addition of a secondary wash.

In these experiments the same parameters described above were evaluated: starting volume(the volume of the cell suspension in the sample bag) and final volume in order to evaluate the washing feasibility of starting sample with small volume of cell suspension; starting and final number of cells in order to evaluate cell loss during thawing and washing steps; the length of the total procedure in order to understand if the procedure is less time-consuming compared to an open system; the volume of the washing buffer used in order to evaluate the reagents consumption.

In the experiments described below, the starting material is a bag of frozen PBL from healthy donor buffy-coat in experiment 4 and a bag of frozen PBL from healthy donor apheresis in the experiment 5, but the procedure may be applied for example to cord blood or bone-marrow samples.

The volume of the starting material is 20-100ml but the range of cell volume to be treated can be between 1ml. to 9999 ml contained in one or more bags.

The number of cells in the bag are approximately 1x10⁹, but the range of cell number to be treated can be between 0.1x10⁹ to 100x10⁹ contained in one or more bags.

Table 2 shows the results obtained in two independent experiments using cells from buffy-coat or apheresis of different donors.

**Table 2**

| | **Experiment 4** | **Experiment 5** |
|---|---|---|
| **Starting volumes** | 20 ml | 100 ml* |
| **Final volume.** | 233 ml | 295 |
| **Starting viable cell number** | 1x10⁹⁺ | 1.2x10⁹⁺ |
| **Final viable cell number** | 0.932x10⁹ | 1x10⁹ |
| **Final cell viability** | 88% | 100% |
| **Cell recovery** | 93% | 83% |
| **Time** | 1 h 20 | 1 h 20' |
| **Thawing buffer** | 1100 ml | 1100 ml |
| **Final volume for cell culture** | 932 ml | 1000 ml |

| | | |
|---|---|---|
| **Viability** = n° of viable cells/n° of total cells x 100, the test is performed using trypan blue staining **Recovery** = n° of final cells/ n° of starting cells 100 *= in this experiment the starting sample was previously thawed and diluted in a bag to 100 ml of thawing buffer. + = the cell number was evaluated the day of cell freezing | | |

### Discussion

The starting volume of the samples in 4 out of 5 experiments is in a range between 15 to 20 ml, only in one experiment the starting sample was diluted with thawing buffer to a total volume of 100 ml.
- The results of these experiments show that there is no major impact of the • starting volume on cell recovery and viability after thawing.
   The mean cell recovery in the first four experiments is indeed 92% with a standard deviation of 5.7% (range: 85-100%) and the value of 83% in the fifth experiment is closed to this range.
   The mean cell viability is 91.5% with a standard deviation of 5.9% (range: 87-100%) and the value of 100% in the fifth experiment is in the range.
   The experiments indicate that the thawing step can be performed starting by small 15-20ml and higher volumes of the starting sample.
- Regarding cell loss, experiments 1 and 3, that were performed testing the starting number of cells after thawing and before cell washing with the CytoMate™, show a maximum cell loss of 9%.
- In the experiments 2, 4 and 5, the number of cells before freezing was considered as the starting cell number. This probably represents an overestimation of the real starting number. Even in that case, a maximum cell loss of 15% was observed.
   These values of cell loss are not significantly different from our current data obtained with an open system (data not shown) and from the data shown in literature using the CytoMate™ (Calmels B. et al., Bone Marrow Transplant. 2003 May;31(9):823-8).
- The length of the total procedure (the steps included are: starting sample thawing, sample washing and sample dilution for cell culturing) is 1 h considering a single cycle of washing with the device, according to the literature data (Calmels B. et al., Bone Marrow Transplant. 2003 May;31(9):823-8), with a delay of 20 minutes considering a secondary washing of the cells.
   Also these data are not significantly different from our current data obtained with an open system that is of about 1h.

According to these results the thawing and washing process performed in a closed system compared to the process performed in a open system is not time-consuming and the safety of the processed cells is well preserved with the closed system because the cells are transferred, during thawing and washing operations, from the initial bag to the final culture bag through a tubing set, the culture medium is added through a bag and samples for cell testing are withdrawn with a sterile syringe. The connections among different bags are made with a sterile tubing welder (Sterile Connecting Device, Terumo) that assures aseptic connections or with the use of spike to be inserted into bag ports.

The system avoids the contact of the sample and of the process reagents with the environment.

### Example 2

### Effect of different lymphocyte stimulations on cell growth, in bags or in flasks.

In order to evaluate cell growth in a closed system, several experiments were done stimulating lymphocytes, in different ways: with OKT^{®} 3 (Orthoclone, Janssen-Cilag S.p.A.) and with Dynabeads^{®} CD3/CD28 T cell Expander (Dynal Biotech, code n° 111.31) both in the presence of recombinant IL-2. Other stimulations can be considered for example with soluble CD3/CD28 antibodies or with different cytokine cocktails.

OKT^{®} 3 is a sterile solution, for clinical use, of murine monoclonal antibody against CD3 antigen. OKT^{®} 3 is currently used in gene therapy clinical studies in order to induce lymphocyte proliferation for cell transduction with retroviral vectors. Retroviral vectors need in fact cell proliferation in order to self-integrate into cell genome during DNA duplication.

Dynabeads^{®}CD3/CD28 T cell Expander are superparamagnetic, polystyrene beads coated with a mixture of CD3 and CD28 antibodies. The CD3 antibody is specific for human CD3 antigen on T cells, as OKT^{®} 3, and CD28 antibody is specific for human CD28 co-stimulatory antigen on T cells.

Dynabeads^{®} mimic in vivo performance of antigen presenting cells with a simultaneous stimulation of T cells with two signals, thus inducing T cell expansion.

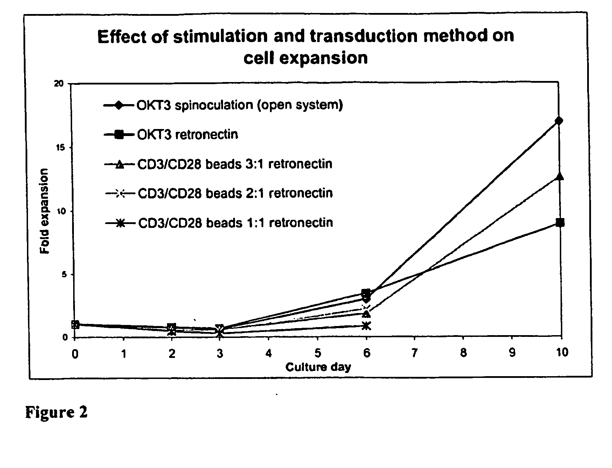

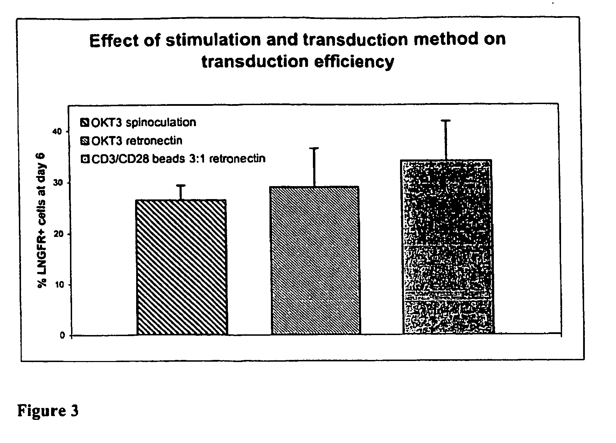

In the following experiments, summarized in figure 2 and 3, PBL from 9 donors were used, in 6 independent experiments, in order to evaluate the effect of OKT^{®} 3 stimulation vs beads stimulation on the cell growth. Cell expansion was determined during 10 days culture in open system (OKT3 spinoculation sample), and in bags (other samples); each point represents the mean of 6-8 data.

The effect of different stimulations on transduction efficiency (% of LNGFR positive cells after transduction) was also evaluated.

In the OKT3 spinoculation sample, the transduction was performed using spinoculation method, in an open system, and in the other samples the transduction was performed using RetroNectin, in a closed system.

These transduction methods and the retroviral vector used for all the experiments whereafter described are extensively explained in example 3 and 4. The vector contains a cell surface marker gene coding for a truncated form of human low-affinity NGF receptor that allows evaluation of transduction efficiency testing ΔLNGFR expression on cell surface with a cytofluorimetric analysis.

Figure 2 shows only preliminary cell proliferation results: all the samples are compared until day 6 but only CD3 versus 3:1 beads are compared until day 10.

In this last step the proliferation differences among the several samples are more evident.

Figure 3 is related to figure 2, the same samples are tested for transduction efficiency comparing the open system to the closed system.

The results, summarized from several experiments in figure 2, demonstrate that the cells stimulated with OKT3 in an open system have a proliferation potentiality higher than the cells stimulated in a closed system. But the stimulation obtained with the use of CD3/CD28 beads increases cell growth in the closed system. These differences in cell proliferation are more evident between day 6 and 10 of cell culture, while until day 6, two or three days after transduction step, cell proliferation is very similar in all samples.

In the same experiments transduction efficiency was evaluated after transduction of lymphocytes stimulated with the different method, figure 3. The results were very interesting showing that stimulation with CD3/CD28 and transduction with RetroNectin in a closed system result in a higher transduction efficiency (% of LNGFR positive cells) than the other stimulation performed with OKT3 either with spinoculation method of transduction or RetroNectin method.

This is very important for the efficiency of the closed system compared to the open system because in spite of less proliferation the final number of genetically-modified cells is higher in the closed system because of the higher transduction efficiency obtained.

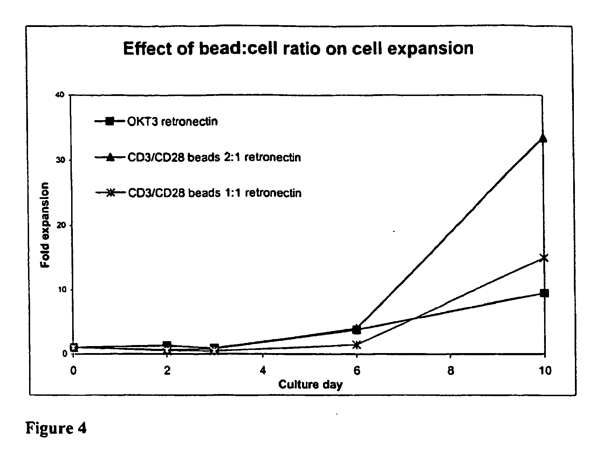

These preliminary results were confirmed with other experiments, a representative of which is showed in fig 3 and 4.

Figure 4 shows a representative cell proliferation experiment that underlines the differences in a closed system among samples stimulated with OKT3 and with two different bead:cell ratios.

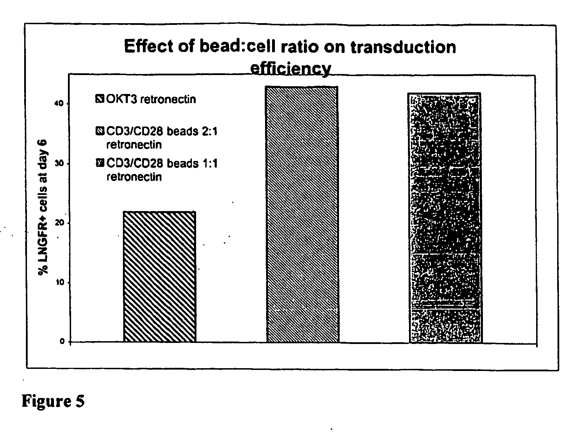

Figure 5 shows instead in the same experiment the transduction efficiency obtained with RetroNectin and spinoculation comparing the data obtained with OKT3 and beads stimulations.

The results confirm the preliminary data obtained with the experiments summarized in figure 3 in which transduction efficiency was higher using RetroNectin than using spinoculation.

Figure 4 confirms that CD3/CD28 stimulation increases cell proliferation in a closed system compared to OKT3 stimulation; and that the beads:cells ratio is very important for cell growth, as at least 2 beads for each cell seem to be required. Probably the 3:1 ratio as suggested by Dynal Biotech could further increase the proliferation rate.

On the contrary (figure 5) this beads:cells ratio doesn't affect the transduction efficiency. Both at 2:1 and 1:1 ratio, the percentage of LNGFR positive cells after transduction is higher than the percentage obtained by OKT3 stimulation, confirming that transduction efficiency after transduction step, obtained in a closed system using RetroNectin is higher with CD3/CD28 stimulation.

These data suggest that genetically-modified cell production in a closed system is very important in order to assure the safety of the product and that it also could be a very efficient system.

### Example 3

### Vectors for cell transduction.

All experiments leading to production of genetically-modified cells for gene therapy were performed using a retroviral vector.

This invention may be used for retroviral vector transduced cells where all the production steps of thawing, washing, transduction, selection, expansion and final recovery can be done in a completely closed system, but it is applicable to other protocols, where only some of these steps are used or the order of the steps has to be changed or inverted, or different vectors are used as lentiviral or adenoviral vectors.

The retroviral vector chosen for the following experiments is SFCMM-3 (Verzeletti et al. HSV-TK gene transfer for controlled GvHD and GvL: clinical follow-up and improved new vectors. Human Gene Therapy, 1998).

The transcription of the ΔLNGFR gene is regulated by the early SV40 promoter.

This vector may be used to produce lymphocytes engineered with the suicide gene HSV-tk for clinical studies in the context of allogenic bone marrow transplantation as explained earlier.

Much data is available about production process of the engineered lymphocytes in an open system. This background was used to compare the open system with the new closed system of the present invention.

### Transduction method transformation changing production process from an open system to a closed system.

The transduction step with retroviral vectors in an open system is performed in clinical studies and also in our open-system method, of genetically-modified cell production, using spinoculation protocol.

In this method the cells, for example lymphocytes, are recovered after 2 or 3 days of stimulation usually with OKT3 under laminar flow boxes by the culture flasks.

The culture medium is washed out with a centrifugation cycle in tubes and the cell are suspended in the retroviral supernatant medium with a concentration between 1 to 5x10⁶ cells/ml of retroviral supernatant, depending on the viral titer.

The cells are plated in plastic vessels for cell culture and centrifuged at 1000-1200 g for 1-2 hours. Then the retroviral supernatant is washed out with cell centrifugation in tubes.

The cell pellet is suspended in culture medium and the treated cells plated in flasks and cultured. Usually a second cycle of transduction is performed after 24 hours of culture.

After 48 hours the cells are tested for ΔLNGFR expression with cytofluorimetric analysis.

In the present invention of genetically-modified cell production in a closed system the transduction step was changed in order to perform washing of the cell and transduction in bags.

The method, extensively explained in example 4 uses the RetroNectin^{®} molecule (Takara), a recombinant human fibronectin fragment, in order to enhance retroviral mediated gene transduction by co-localizing target cells and virions. Culturing of the cells with retroviral supernatant in RetroNectin^{®}-coated bags for 12-24 hours allows cell transduction in a closed system.

This transduction method was compared to spinoculation method after lymphocyte stimulation with OKT3 in several experiments, already summarized in example 2, figure 3.

In the table below, table 3, the characteristics of the two different transduction method are summarized.

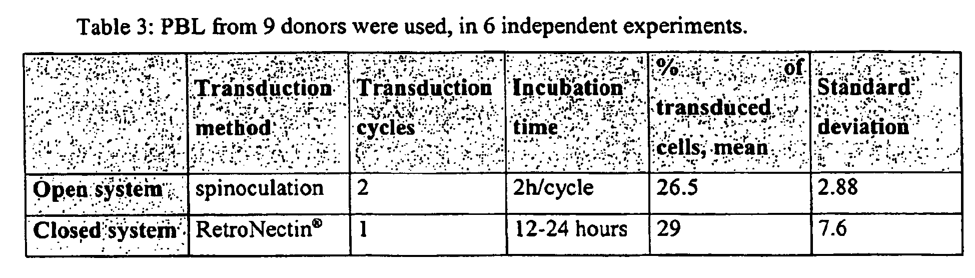

The advantages of using RetroNectin transduction method are: the passage to a closed, more safe system, the reduction of transduction cycles from 2 to 1, the elimination of the potentially dangerous centrifugation step and the increase in transduction efficiency.

Safety considerations and improved efficiency results led us to use the RetroNectin^{®} transduction method in the development of the closed system production process.

### Example 4

### Lymphocytes transduction.

The second step after cell stimulation is cell transduction.

In the following experiments the lymphocytes transduction was performed after a stimulation step with OKT3 (Orthoclone) (but different protocols of stimulations can be applied as described in the previous paragraph), culturing the cells for 24h with retroviral supernatant (the vector is SFCMM-3, previously described, containing the gene of interest and a gene for a cell surface marker) into RetroNectin^{®} (Takara) pre-coated bags. RetroNectin^{®} is a recombinant human fibronectin fragment that enhances retroviral mediated gene transduction by co-localizing target cells and virions.

The transduction is generally performed in a range between 0 to 7 days after stimulation. In the experiments described below, transduction was performed on day 2.

The transduction process using RetroNectin^{®} involves different steps: preparation of RetroNectin^{®} coated bags, preparation of target cells, loading of retroviral vector on the RetroNectin^{®} pre-coated bags, incubation of the cells into pre-loaded bags.

The RetroNectin^{®} lyophilized powder is dissolved in sterile water for injection, an appropriate volume of this solution is dispensed into each bag (CellGenix Vuelife bags or Baxter x fold bags) and incubated at room temperature (RT) for 2 hours, protected by light. The RetroNectin^{®} concentration used is generally in a range between 1 to 8 µg/cm² of bag surface. In these experiments 1.2 µg/cm² were used.

After removing RetroNectin^{®} solution, a sterile solution of PBS 2% Human Serum Albumin (HSA) is added to each bag for blocking. A 30 minutes incubation at RT is required, then the HSA solution is removed by three washing with PBS: The bags can be used immediately, preserved at 4°C or at -80°C for up to a week.

The retroviral supernatant is then pre-loaded to the RetroNectin^{®} in the bags during 1 h incubation at 37°C.

The volume of retroviral supernatant to be used for cell transduction is related to the cell number and the ratio between cell number and supernatant volume may be in the range between 0.5x10⁶ cells/ml to 10x10⁶ cells/ml.

The supernatant is supplemented with IL-2 (range: 100-600 IU/ml), 2mM glutamine and 3% autologous plasma
In these experiments the ratio of 1x10⁶ cells/ml of retroviral supernatant is used. Regarding the pre-loading step, the range of supernatant to be used is between 0 and the total volume planned. In these experiments the supernatant used for the pre-loading step was half of the planned.

Just prior to infection, the cells are collected and then suspended in the remaining volume of supernatant, reagents added as described above. As mentioned above the final ratio of 1x10⁶ cells/ml of retroviral supernatant was used.

The cell incubation in the bags was performed for 24h, but may be, for example, in the range of 1 h to 72h.

Between transduction and gene expression there is usually a delay of 24-48h, in this step in fact the ALNGFR protein is not already detectable.

The data showed in table 3 summarize the results on transduction efficiency tested 48h after transuction process.

### Transduction using CyloMate™

As described in detail in example 1, one aspect of the present invention provides a method for cell manipulation and culturing in a completely closed system with the use of a device for cell manipulation in clinical grade closed disposable kits.

In this example the CytoMate^{™} was used as an automated fluid management device in order to collect the cells just prior to infection.

According to the protocol described above, before transduction, one or more bags are pre-coated with RetroNectin^{®} and pre-loaded with half volume of the retroviral supernatant planned.

On day 2 after OKT3 stimulation the bag (or bags) of cultured lymphocytes are collected using CytoMate and suspended with half volume of the planned supernatant in the final preloaded bag (or bags). The final ratio of 1x10⁶ cells/ml of retroviral supernatant was used.

The incubation of the cells in the bags was performed for 24h.

Cell washing and concentration are performed as for the thawing of the cells in a sterile disposable set consisting of the spinning membrane connected to a filtered wash bag, a buffer bag and a waste bag. The sample bag with the cultured lymphocytes and the final bag with the pre-loaded supernatant are aseptically connected to the set. This system allows for cell processing in a GMP environment.

The washing procedure is defined by the user and is different in different steps of the genetically-modified cell production process.

Briefly cells are washed with 2 cycles using cold buffer (X-Vivo 15, glutamine 1% and IL-2 600 IU/ml added) with the use of the spinning membrane and transferred into a collection bag, the wash bag. The cells are then transferred to the final bag in a small volume of about 40ml, the wash bag and tubing are rinsed with half of the supernatant planned in order to reducing cell loss. This supernatant is transferred to the final bag and the cells are diluted to 1x10⁶ cells/ml of supernatant.

The retroviral supernatant in the final bag is diluted by the 40ml, that depends on different defined parameters, of the washing buffer but this dilution doesn't lower the transduction efficiency of the retroviral supernatant (data not shown).

The efficiency and length of the washing step depend on the selected parameter "residual fold reduction". The value of 50 was chosen on the basis of Nexell data (CytoMate^{™} Custom Programming Guidelines). The value of 100 will result in approximately 2 log reduction of the source solution.

At the end of the washing procedure a sample of cells can be evaluated for number of viable cells and the cells are finally incubated for 24h in an incubator at 37°C and 5% CO₂. The supernatant is a reagent where the infective retroviral particles are resuspended in X-VIVO 15, the cell culture medium. As described above the supernatant is added before transduction with glutamine 1%, IL-2 600 IU/ml, autologous plasma 3% and protamine. This culture environment allows the cell culturing during the 24h of incubation with the retroviral particle.

The length of the washing procedure including dilution is of the order of 1 h.

Three different experiments were conducted using the washing procedure described above. In these experiments different steps were evaluated: starting volume (the volume of the cell suspension in the sample bag) and final volume; starting number of cells in order to evaluate the supernatant volume to be used (1x10⁶ cells/ml of supernatant), final number of cells in order to evaluate the final ratio "cells versus supernatant"; the length of the total procedure in order to understand if the procedure is less time-consuming compared to an open system; the volume of the washing buffer used in order to evaluate the reagents consumption.

The table 4 summarize the data of the three washing experiments performed using CytoMate™.

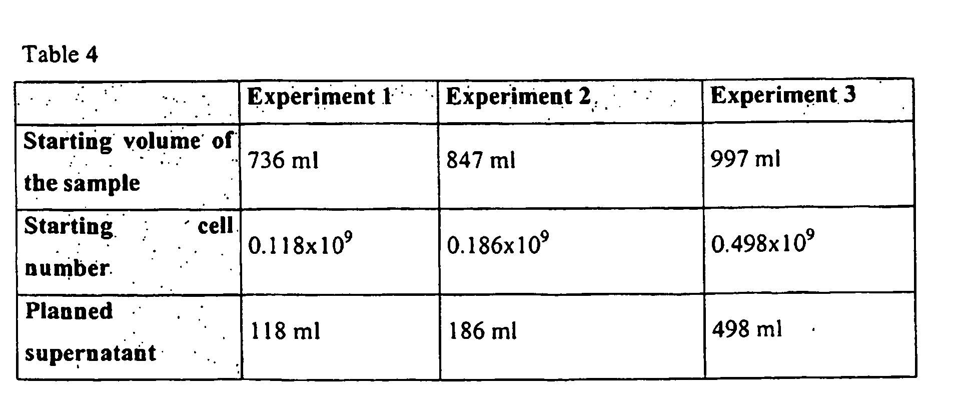

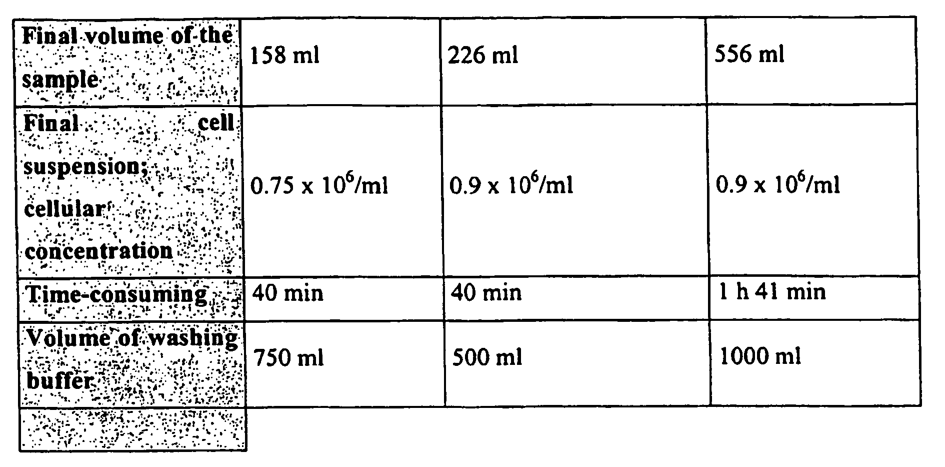

The starting volume of the samples depends on the number of the cells at the day of stimulation when the cells are cultured at a concentration of 1x10⁶/ml. The number of cells depends on the stimulation and using OKT3, the number usually decreases within two days after stimulation. With knowledge of the cell number, the volume of supernatant to be used can be defined using the ratio 1x10⁶ cells/ml of supernatant. The final volume of cellular suspension is the result of the volume of the suspension after washing (about 40 ml of washing buffer, X-Vivo 15) plus the added volume of supernatant. In this respect, the final supernatant is diluted at a maximum of 0.33. This dilution doesn't lower the transduction efficiency of the supernatant. The cells are concentrated during the transduction step in a range between 0.75-0.9x10⁶/ml and this concentration allows a good gene transfer into the cells as described in the next paragraph.

The procedure with CytoMate^{™} is very short. Only in experiment 3 was it longer than 1 hour. The use of this RetroNectin protocol in a closed system allows the transduction cycle to be reduced to 1, while in an open system with spinoculation there are two cycles taking two hours.

According to this data, the washing process performed in a closed system in order to prepare the cellular suspension for transduction is fast and the safety of the processed cells is well preserved. This is because the cells are transferred, during washing operations, from the initial bag to the final RetroNectin pre-loaded bag through a tubing set, the supernatant is added through a bag and samples for cell testing are performed with a sterile syringe (cell counting). Thus, the system avoids the contact of the sample and of the process reagents with the environment. This is in contrast to the transduction cycle using spinoculation where the cells are manipulated in flasks under a laminar flow box in class A.

### Example 5

### Lymphocytes culturing after transduction

After 24h incubation with the retroviral supernatant the cells were harvested and suspended in culture medium in bag (or bags) for cell culture. The culture medium is the same used for stimulation: X-Vivo 15 supplemented with autologous plasma 3%, glutamine 1% and IL-2 (range: 100-600 IU/ml).

In this example the CytoMate^{™} was used as an automated fluid management device in order to manipulate the cells in a closed system. In this step the cells are washed using X-Vivo 15 and suspended in culture medium and the retroviral supernatant is discarded.

A fraction of the cultured cells are now genetically modified, using the retroviral vector described in example 3 containing the gene of interest and a marker gene, a truncated form of the NGF receptor (ΔLNGFR). After some days of culture, in this protocol two days, the cell population is tested for ΔLNGFR expression. The gene coding for ΔLNGFR is not endogenously expressed in leucocytes but only in transduced cells and is detectable on the cell surface by FACS (Fluorescence Activated Cell Sorter) analysis with a fluorochrome coupled anti LNGFR antibody.

Briefly the fluorochrome conjgated cells pass through a laser beam. They disrupt and scatter the laser light, which is detected as forward scattered and side scattered light. The first parameter is related to cell size while the second is an indicator of a cell's internal complexity. In addition to scatter, the cytometer measures fluorescence parameters. The fluorochromes absorb the laser light and emit a portion of this absorbed light in different regions of the spectrum. The cytometer measures the relative amount of each dye on individual cells, processes the electronic signal resulting from each cell and creates numeric values for each parameter. It then passes the information to the computer system for display and analysis. The fluorescence intensity is proportional to the amount of fluorochrome conjugated antibody bound to the cell surface.

### Washing out retroviral supernatant using CytoMate™

In this step the cell washing and concentration are performed as described in example 4 in a sterile disposable set consisting of the spinning membrane connected to a filtered wash bag, a buffer bag and a waste bag. The sample bag with the transduced lymphocytes and the final bag for cell culturing are aseptically connected to the set. As previously mentioned this system allows for cell processing in a GMP environment.

The washing procedure is defined by the user and is the same as described in example 4. Cells are washed with 2 cycles using buffer (X-Vivo 15, plus glutamine 1% and IL-2, range: 100-600 IU/ml) with the use of the spinning membrane and transferred into a collection bag, the wash bag, the cells are then transferred to the final bag in a programmed volume, the wash bag and tubing are rinsed with a fixed volume of the buffer in order to minimize cell loss.

The cell suspension in the final bag is then diluted according to the final cell number in order to culture the cell with a concentration of 0.2-0.5x10⁶ cells/ml of culture medium. Autologous plasma was added at 3% of the total volume.

The efficiency and length of the washing step depend on the selected parameter "residual fold reduction". A value of 50 was chosen on the basis of Nexell data, CytoMate^{™} Custom Programming Guidelines. A value of 100 will result in approximately 2 log reduction of the source solution.

The length of the washing procedure including final dilution is approximately 1h.

Three independent experiments were conducted using the washing procedure described above. In these experiments the following parameters were considered: starting volume, starting number of cells, the length of the total procedure and the volume of the washing buffer used in order to evaluate the reagents consumption. In one experiment the final number of cells was evaluated in order to test cell loss during the washing step.

The table 5 summarizes the data of the three washing experiments performed using CytoMate™.

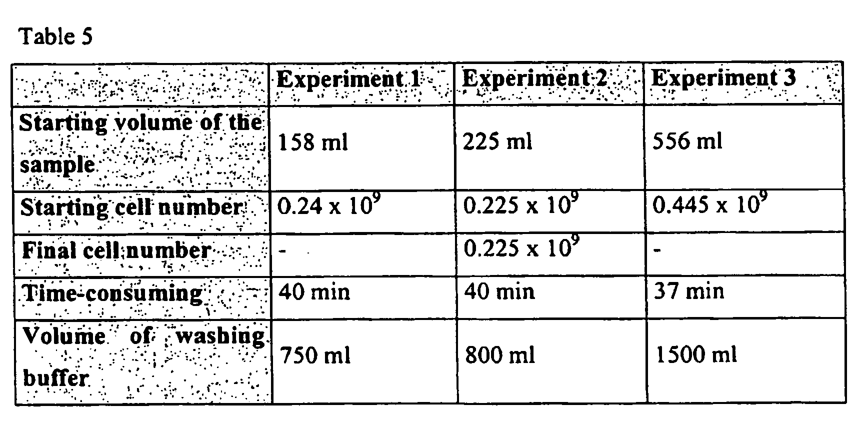

The starting volume of the samples depends on the number of the cells at the day of transduction when the cells are cultured 1x10⁶/ml in retroviral supernatant. From the starting cell number, the volume of culture medium to be used can be defined using the ratio of 0.5x10⁶ cells/ml of culture medium.

The procedure with CytoMate^{™} is very short as described also in example 4.

Using an open system the cells should be centrifugated in order to discard the supernatant and under a laminar flow box have to be diluted with culture medium in flasks for cell culturing. This step causes a physic stress to the cells and operational difficulties because the volume to be manipulated could be very high. For example in experiment 3, 556 ml of retroviral supernatant has to be removed and 1100 ml of culture medium has to be distributed in many flasks. These operations augment the risk of product contamination.

The time used in an open system for these operations is about 1h and 30 minutes according our data.

According to this data the washing process performed in a closed system in order to prepare the cellular suspension for cell culturing compared to the washing process performed in a open system is less time-consuming; the safety of the processed cells is well preserved with the closed system because the cells are transferred, during washing operations, from the initial bag to the final culturing bag through a tubing set, the culture medium is added through a bag and samples for cell testing are performed with a sterile syringe (cell counting).

This system avoids the contact of the sample and of the process reagents with the environment and eliminate the centrifugation stress that can affect the viability of cells.

### Example 6

As described in the previous example, during two days of culture, after the transduction step, the genetically-modified subpopulation, express the ΔLNGFR cell surface molecule. This molecule allows for genetically-modified subpopulation selection using a magnetic beads system.

The cell suspension is incubated with a GMP-grade mouse IgG anti NGF receptor antibody that binds to the ΔLNGFR positive cells, the genetically-modified subpopulation. After two washing steps a second incubation with GMP-grade immunomagnetic beads coated with sheep anti-mouse IgG (Baxter, code RAR 9950) is performed.

The beads treated cell suspension is now applied to a magnet in order to isolate ΔLNGFR positive cells, coated by beads. The negative population, that is not captured to the magnet is washed out with the adequate buffer (PBS 0.1% HSA).

The positive cells are then recovered in culture medium switching off the magnet. After 24h of culturing the binding between antigen and antibody is disrupted. The beads are released in the medium and removed with the use of the magnet. During the application of the magnet, the beads bind the magnet and the cell population, that is not captured by the magnet is washed out with the adequate buffer (PBS 0.1 % HSA).

The recovered cell population, a homogeneous genetically-modified population, is cultured for some days, in a range between 1 to 7 days, and tested for cell proliferation and ΔLNGFR expression after 48h minimum.

As described for the transduction step, after down regulation of the ΔLNGFR molecule, the gene coding for ΔLNGFR molecule needs 24-48h for molecule expression on cell surface.

The cytofluorimetric assay used to test ΔLNGFR expression was described in the previous example.

### Antibody incubation and cell washing using CytoMate™

The present disclosure uses *CytoMate™* in order to perform the following steps in a closed system: washing out of the culture medium and cell concentration, incubation of cell suspension with the anti NGF receptor antibody, washing out the exceeding antibody and concentration of the cells for beads incubation.

Washing and concentration are performed as described in example 4 in a sterile disposable set consisting of the spinning membrane connected to a filtered wash bag. A buffer bag and a waste bag are also connected to the set. The buffer used for incubation step is PBS 0.5% HSA, the one used for washing steps is PBS 0.1% HSA.

The sample bag with the transduced cells suspended in culture medium and the final bag for cell recovery are aseptically connected to the set. The cells are tested for cell number before washing in order to plan the selection operations.

As previously discussed this system allows for cell processing in a GMP environment. Cells are concentrated in the wash bag with a programmed 1 cycle of washing with the use of the spinning membrane, the previously described buffer for washing out of medium culture is used.

The antibody solution with the planned micrograms of antibody, 1ug/5x10⁶ cells, was added to the wash bag. The "Circulate Wash Bag" step previously planned after the washing step, circulates the cell suspension in the wash bag trough the wash circuit. This operation mix the cell suspension with the antibody solution for 20 minutes according to the program set by the user.

After the incubation time the exceeding unbound antibody is washed out with a second cycle of washing and the cells are then transferred to the final bag in a programmed volume and the wash bag and tubing are rinsed with a fixed volume of the buffer in order to reducing cell loss.

The antibody bound cell suspension collected in a bag is now ready to be incubated with the beads using a different device equipped with a magnet.

The efficiency and length of the washing steps depend on the selected parameter "residual fold reduction". The value of 50 was chosen on the basis of Nexell data. The value of 100 will result in approximately 2 log reduction of the source solution.

The length of the whole procedure of cell washing and cell incubation with the antibody is of the order of 1h and 30 minutes.

Three different experiments were conducted using the procedure just described. In these experiments the following parameters were considered: starting volume (the volume of the cell suspension in the sample bag), final volume of cell suspension in order to concentrate the cell for the subsequent incubation step, starting number of cells in order to plan the reagents for the selection procedure, the length of the total procedure in order to understand if the procedure is less time-consuming compared to an open system and the volume of the washing buffer used in order to evaluate the reagents consumption. In one experiment the final number of cells and viability were tested in order to evaluate cell loss during the procedure.

The table 6 summarize the data of the three experiments performed using CytoMate™.

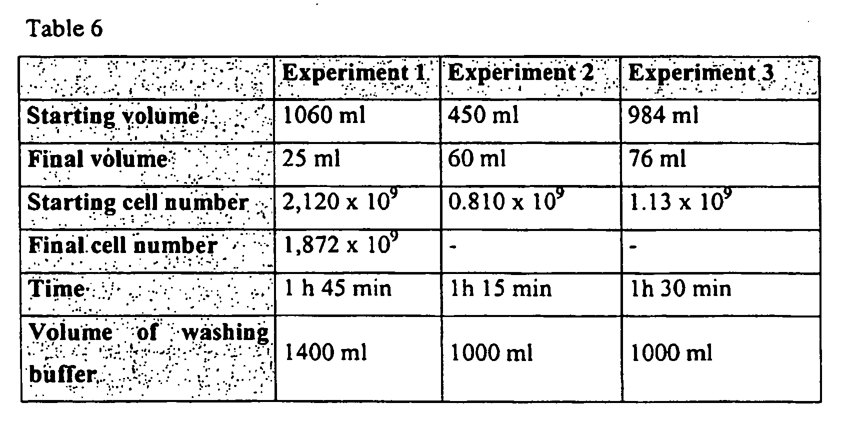

The starting volume of the samples depends, on the number of the cells after the transduction step (see example 5), when the cells are cultured 0.5x10⁶/ml in culture medium.

The final volume of the cell suspension has to be detected in order to concentrate the cells for the subsequent step of incubation with the beads.

With the starting cell number the volume of incubation buffer (to prepare the antibody solution, 20x10⁶ cells/ml of buffer) and the micrograms of antibody to be used (1µg/5x10⁶ cells) can be defined.

The final cell number and cell suspension viability were tested in one experiment in order to evaluate cell loss during the procedure. The viability is 100% showing that the procedure doesn't stress the cells.

Cell recovery was, in experiment 1, 88% indicating that there is a small cell loss after CytoMate™ procedure that could be reduced by changing the program, for example reducing the washing step.

The procedure with CytoMate™ is not time consuming and is about 1h and 30 min. Many operations are done in this period of time: 2 washing and incubation of 20 min. The time used in an open system for these operations is about 2h according our experience.

According to this data, the washing, concentration and incubation process performed in a closed system in order to bind the cellular suspension with the antibody and to prepare the cells for magnetic selection compared to the same process performed in a open system is less time-consuming and the safety of the processed cells is well preserved with the closed system because the cells are manipulated using a tubing set, the reagents are added through a bag and samples for cell testing are performed with a sterile syringe (cell counting).

This system avoids the contact of the sample and of the process reagents with the environment and eliminate the centrifugation stress that can affect the vitality of cells.

### Immunomagnetic selection

The final product of the previous process, the sensitized cells (incubated with the anti NGF receptor), is now concentrated in a bag. The bag can be aseptically connected to a second tubing set in order to start the selection step using paramagnetic microspheres (beads) and a device equipped with a magnet (a magnetic cell separator). The sterile, non pyrogenic disposable set is composed of a vented cylindrical 150ml chamber. The chamber is permanently connected in the bottom through a Y connector with two tubing systems: the inlet line and the outlet line. Both lines have two spike connections for attaching bags containing the cellular suspension (the sample bag) and working buffer to the chamber through the inlet line and for attaching the final cell collection and waste bags to the chamber through the outlet line.

In the experiments described hereafter the magnetic separator used is Isolex™ 300 designed by Baxter International Inc. which is used to select and isolate CD34+ cells from a heterogeneous cell population utilizing an anti-CD34 monoclonal antibody and paramagnetic microspheres.

In the Isolex™ 300 standard protocol the cells are sensitized with the anti-CD34 monoclonal antibody in a open system, then the cells are mixed with the beads using a tubing set, the CD34+ cells magnetically isolated and the unbound negative cells washed out. Finally the CD34+ cells are released from the beads using a peptide molecule with high affinity for the primary antibody. The antibody/peptide complex can be magnetically held within the separation chamber while the CD34+ cells are released into a collection bag.

This process was followed for our purposes only for mixing the sensitized cells (incubated with the anti NGF receptor) with the beads (Baxter, as described in detail above) and for magnetic isolation of the rosetted ΔNGFR positive cells. The unrosetted negative cells are washed out from the separation chamber to a waste bag with three washing cycles.

Briefly the two bags of the inlet line and the two bags of the outlet line are aseptically connected with a sterile tubing welder (Terumo) that assures aseptic connections or with the use of the spikes of the lines to be inserted into bag ports. This system avoids the contact of the sample and of the process reagents with the environment. After the connections, the fixed set program starts with this sequence of steps: priming of the tubing set with the washing buffer, mixing of sensitized cells in the chamber with the beads, added before incubation into the starting cell bag, magnet separation of rosettes, washing off residual beads and negative cells.

The washing buffer is PBS 0.1% HSA. The beads are added according to the starting cell NGF positive number in a ratio 5 beads to1 NGF positive cell. The number of NGF positive cells is detected before selection in a small amount of cellular suspension counting the cells and testing the percentage of NGF positive cells by FACS analysis, as previously described in example 5.

A volume of 100ml is not exceeded - 90ml of the starting solution and 10ml of the pre-washed planned beads. A maximum of 10x10⁹ sensitized cells can be treated with this device. The time of incubation of the mix is 30 min. at room temperature.

After mixing the rosetted NGF+ cells are retained on the primary chamber wall by the magnet. The unrosetted cells are washed out in the waste bag and then the NGF+ cells are washed three times with the buffer in order to remove residual unrosetted cells. The working buffer used for washing is collected together with the unrosetted cells and this cell suspension can finally be counted in order to control the good outcome of the selection procedure.

Two different experiments were conducted using the procedure described above. In these experiments the following parameters were determined: starting volume (the volume of the cell suspension in the tubing set chamber), final volume of cell suspension in order to concentrate the ΔLNGFR+ cells for culturing after immunomagnetic separation, percentage of NGF+ cells tested by FACS analysis, total number of ΔLNGFR+ cells, derived from the starting cell number (detected before CytoMate™ washing) and percentage of ΔLNGFR+ cells, the length of the total procedure in order to understand if the procedure is less time-consuming compared to an open system, the number of cells suspended in the waste bag and the volume of the washing buffer used in order to evaluate the reagents consumption.

The table 7 summarize the data of the two experiments performed using Isolex™ 300.

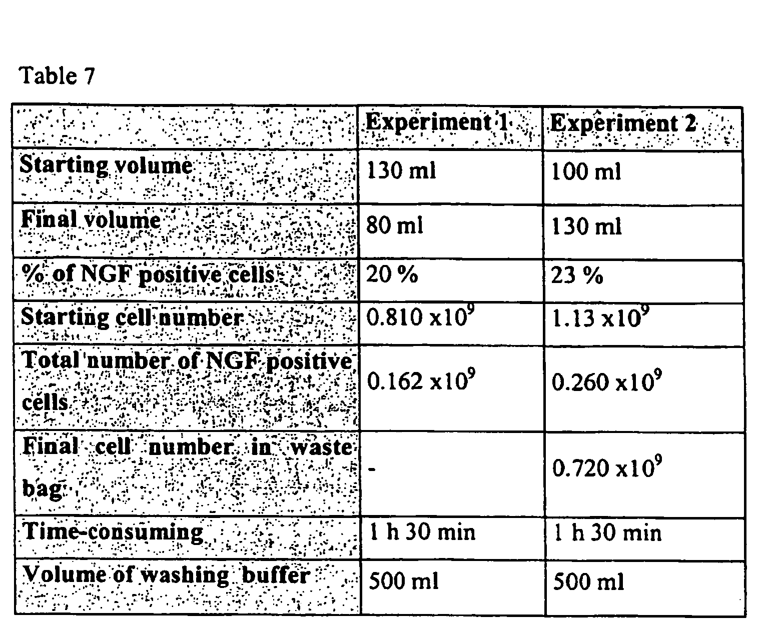

### Discussion

The starting volume of the samples depends on the washing and sensitization steps performed with CytoMate™ that wash about 80 ml plus the added volume of pr-washed beads, the total volume being about 100 ml. A volume of 130 ml has not to be exceeded because of the volume of the tubing set chamber is 150 ml. 20 ml of residual volume is needed for a good mix between cells and beads.

The final volume of the cell suspension is fixed on the basis of the total number of LNGFR positive cells. The volume of cell suspension is the volume of cell culture and the cells are plated with a concentration of 2x10⁶ LNGFR positive cells/ml of culture medium. After washing the rosetted cells, a bag with the planned volume of culture medium is aseptically connected with a sterile tubing welder (Terumo) that assures aseptic connections.

The percentage of LNGFR+ cells is related to the transduction step and depends on different factors including: stimulation of the cells, transduction method, viral titer of the supernatant used for transduction and number of transduction cycles.

A higher percentage of LNGFR positive cells is very important because it improves the recovery of the final genetically-modified cells and the efficiency of the whole procedure.

The total number of LNGFR positive cells related to the starting cell number and to the percentage of LNGFR positive cells is important in order to prepare the reagents for selection, the number of beads and working buffer to be used, and in order to evaluate the recovery of the cell after selection (i.e. the efficiency of the selection step). Cell recovery is evaluated the next day after selection when the down-regulation of the ΔLNGFR molecule releases the beads in the culture medium. The cells are tested for cell number using tripan-blue staining. These results are shown in table 8 and it is, as extensively discussed in example 8, within the mean of the recovery results obtained in an open system.

The final cell suspension was counted in one of the two experiments in order to assess the outcome of the selection procedure. Less than 10% of the total cells are lost during procedure.

The selection procedure is not time consuming compared to an open selection method in which the cell suspension has to be divided in at least 3 tubes and each tube has to be applied to a magnet. Also the next washing steps on the rosetted cells has to be performed in several tubes and each tube applied to a magnet which is time consuming. Moreover, manipulation of several tubes under laminar flow box is associated with a high risk of contamination compared to the manipulation of the cells in the tubing set.

### Immunomagnetic microspheres detachment

The Dynabeads® (Baxter) M-450 Sheep anti-Mouse IgG are paramagnetic, polystyrene beads with affinity purified sheep anti-Mouse IgG covalently bound to the surface. The sterile non-pyrogenic suspension is for ex-vivo use only. For this reason the beads have to be removed from the cell suspension. As described above, the day after selection, the rosetted cells release the beads in the culture medium. Then the cell suspension is treated with the magnetic cell separator system in order to remove the beads from the cell culture.

A tubing set with a chamber is used as described in the previous section.

Briefly the two bags of the inlet line and the two bags of the outlet line are aseptically connected with a sterile tubing welder (Terumo) that assures aseptic connections or with the use of the spikes of the lines to be inserted into bag ports. The connected bags of the inlet line are: the buffer bag (the buffer is the medium of culture) and the cell suspension bag. The connected bags of the outlet line are: the final cell suspension bag, for cell recovery, and the waste bag.

After connections the fixed set program starts with this sequence of steps : priming of the tubing set with the washing buffer, mixing of sensitized cells in the chamber with the beads, added before incubation into the starting cell bag, magnet separation of rosettes, washing off residual beads and negative cells.

Only the set of washing is used for our purposes: the beads are retained on the chamber wall by the magnet while the cells are collected in the final bag.

Two di fferent experiments were conducted using the procedure described above. The selection step of the cells of these experiments is described in the above section.

In these experiments the cell recovery was evaluated in order to compare selection and detachment of the present invention performed in a closed system to selection and detachment performed in an open system.

Table 8 summarizes the recovery of the cells after bead detachment performed using Isolex™ 300.

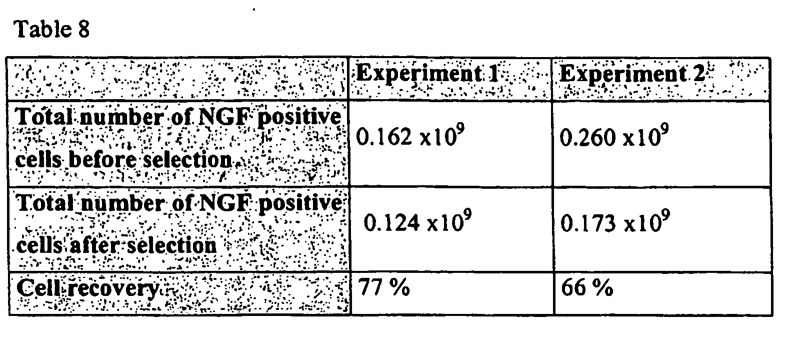

As extensively discussed in example 8, the results of cell recovery in experiment 1 and 2, in which selection and detachment are performed in a closed system, are within the mean values of results obtained for selection and detachment performed in an open system.

### Example 7

### Lymphocytes culturing after selection

After beads detachment the cells are cultured for 24 hours. The cells are counted using trypan blue staining and ΔLNGFR expression is evaluated by FACS analysis, in order to test the homogeneity of the selected population.

The cells cultured in bags are then diluted in culture medium at a concentration range between 0.2 to 1x10⁶ cells/ml, adding the medium using a sterile tubing welder (Terumo) that assures aseptic connections among bags.

Usually, the ΔLNGFR molecule is already expressed in more than 90% of the population. However, depending on cell proliferation rate, it may be that not all the cells express the cell surface molecule at this time resulting in a non-homogeneous population. In any case, the same cytofluorimetric analysis is repeated the last day of culture before patient infusion or cell freezing in order to confirm that the cell population is completely genetically-modified.

The post-selection days of culture are in a range between 0 to 6 depending on the number of cells planned in the clinical study for each patient infusion; an aliquot of the population is used for quality control analyses, that include sterility test.

According to the European Pharmacopoeia (EP), an aliquot of the product is tested; the volume of the aliquot depends on the total volume of the product. On the basis of statistical considerations, it can be assumed that if this aliquot doesn't give rise to bacterial growth, when tested in the sterility test, the whole product can be considered as sterile.

However, the presence of contaminants at extremely low charge in the final product cannot be absolutely excluded. This risk can be minimized by using a cell culture system such as the present invention, whose major advantage is cell manipulation in a completely closed system.

In the present case, the safety of the final product is assured by the system itself since, in each step of the production, the cells are never in contact with the environment.

The cells are always manipulated in sterile tubing sets and treated with reagents prepared in sterile bags; thus, the aseptically environment is very easily assured.

### Final product washing and recovery using CytoMate.

In this step the final product is recovered, washed in order to eliminate the culture medium and then concentrated in 50-60ml of final volume in order to be infused into patient or frozen for a future infusion.

In the following experiments the buffer used for cell washing is PBS added with 0.5% HSA, but for a clinical protocol of gene therapy a sodium chloride 0.9% solution with 0.5-4% HSA has to be used in order to better preserve a physiological environment for the cells and in order to prepare a physiological product, suitable for infusion in the patient.

Washing and concentration steps are performed as described in example 4 in a sterile disposable set consisting of the spinning membrane connected to a filtered wash bag, a buffer bag and a waste bag. The sample bag with the genetically-modified lymphocytes and the final bag for cell recovery are aseptically connected to the set.

As previously mentioned, this system allows for cell processing in a sterile environment.

The washing procedure is defined by the user and is the same as described in example 4. Cells are washed with 2 cycles using the buffer just described, with the use of the spinning membrane and transferred into a collection bag, the wash bag. The cells are then transferred to the final recovery bag in a programmed 50-60 ml volume and the wash bag and tubing are rinsed with a fixed volume of the buffer in order to reducing cell loss.

As described above, the final product, the genetically-modified cells, in the final recovery bag, can be suspended in saline solution supplemented with HSA 0.5-4% for infusion or with HSA 0.5-4% and DMSO 10% for cell freezing.

Before infusion or freezing an aliquot of the final product is controlled for quality with the tests required by European regulations.

The efficiency and length of the washing step depend on the selected parameter "residual fold reduction"; the value of 50 was chosen on the basis of Nexell data, the value of 100 will result in approximately 2 log reduction of the source solution.

The length of the washing and concentration procedure is of approximately 1h.

Two independent experiments were conducted using the washing procedure just described. In these experiments, the following parameters were analyzed: starting volume (the volume of the cell suspension in the sample bag), starting number of cells and final number of cells in order to test cell loss during the washing step, the volume of the washing buffer used in order to evaluate the reagents consumption and the length of the total procedure. The last point is very important in this step, in which the viability of the cells is a fundamental issue.

Table 9 summarize the data of the two washing experiments performed using CytoMate™.

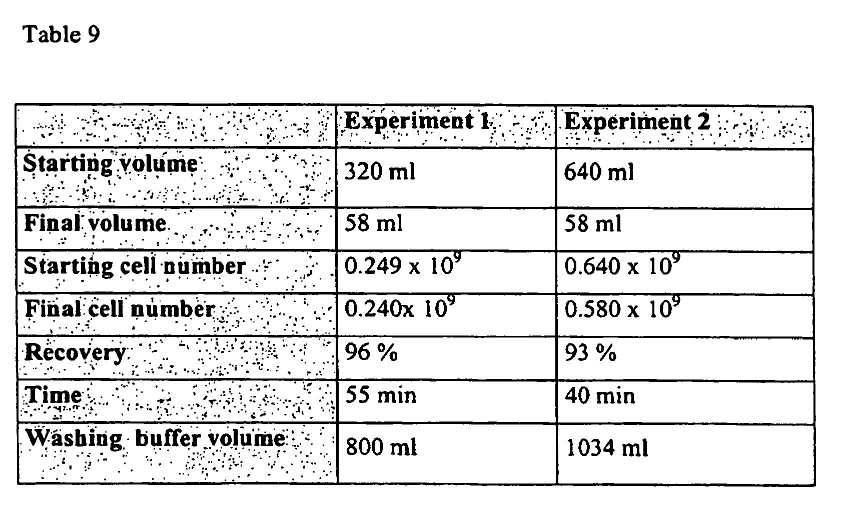

The starting volume of the samples depends on the number of the cells at the previous step of splitting when the cells are cultured at 0.2-1x10⁶ cells/ml of culture medium.

The final volume of cells in the recovery bag is set in the program by the user and preferably does not exceed 60ml in order to minimize the volume to inject into the patient.

The starting and final number of cells is very useful data in order to evaluate cell loss during the procedure and for clinical studies purposes in order to plan the dose to be injected to the patient usually defined, in gene therapy clinical studies (Bibliografia), as number of cells/Kg of patient weight.

As described in example 4, the procedure with CytoMate^{™} is very short.

In this step in which the final recovered cells are the final product, the total time of cell manipulation has to be minimized in order to preserve cell viability until patient injection or cell freezing. Stability tests, performed in our laboratories, on the viability of the final product suspended in sodium chloride 0.9% with 4% HSA indicate that the cell suspension maintains the stability for four hours: thus 1h is an adequate time for this process step.

The viability of the product was evaluated and was found to be 96-97% in the two experiments, while cell loss during the washing step was 4-7%. This result assures the quality of the final product for patient injection or freezing.

According to this data, the washing process performed in a closed system in order to prepare the final product, the genetically-modified cells for injection or for freezing, is not time-consuming and the quality and the safety of the processed cells are well preserved: the cells are transferred, during washing operations, from the initial bag to the final infusion or freezing bag through a tubing set and the control of product quality is performed taking away an aliquot of the product with a sterile syringe.

### Examples 8

### Comparison between open and closed systems

As extensively explained in the previous examples the present invention provides a method for transduction and selection of genetically modified cells in a standardized safe closed system for clinical use.

In particular, the present invention was used (see previous examples) for the production of genetically modified T cells to be used in the context of allogeneic bone marrow transplantation clinical protocols.

Currently an open system is used for production of transduced T cells for clinical protocols. The steps of this system are summarized above and include: stimulation, transduction, selection, expansion and harvest.

In each example a single step was described together with the development of the step into a closed system.

The transformation from an open system to a closed system is necessary for the scale-up of the product and it is necessary in order to improve the safety of the product for clinical use.

In our experience an open system may be used, without significant risk of contamination, if limited number of cells are required (1-1.2x10⁸ of final cells, patient dose 10⁶ cells/Kg).

But when the clinical protocol require high doses (10⁷ or 10⁸ cells/Kg), 0.8-8x10⁹ final cells are produced and a closed system is necessary in order to reduce contamination risk and to facilitate cell manipulation.

With this purpose an easily applicable closed system was developed in our laboratories. As explained in each step described above, this system assures the aseptical manipulation of higher amount of the cells compared to an open system. As no open steps are present, the whole manipulation may be performed in conventional laboratories.

The method can be standardized in protocol devices in order to be used by a wide range of laboratory operators.

In the table below two experiments extensively discussed in the previous examples are summarized and compared step by step with experiments conducted in an open system.

Each value of the open system is the media of 5 experiments conducted using 5 different donor cells recovered by apheresis, while the two experiments performed with the closed system were performed starting by a buffycoat and an apheresys.

**Table 10**

| | **Day2/Day0** | **Day3/Day2** | **Day6/Day3** | **%NGF MFI before set** | **MFI before selection** | **% recovery** | **Day10/Day7** | **final % NGFR** | **final % NGFR** | **Day10/Day0** | **N° of final cells x 10e6** |
|---|---|---|---|---|---|---|---|---|---|---|---|
| **Open system (5 donors)** | 0,41 | 1,3 | 3,7 | 15 | 507 | 34 | 5 | 95 | 580 | 0,35 | 568 |
| **Closed system (buffy-coat)** | 0,2 | 1,2 | 3,6 | 20 | 622 | 77 | 2 | 96 | 519 | 0,26 | 249 |
| **Closed system (apheresis)** | 0,49 | 0,89 | 2.5 | 23 | 364 | 67 | 3,6 | 94 | 462 | 0,64 | 640 |

- Day2/Day0 shows the ratio between cell numbers tested at day 2 and day 0. This ratio indicates cell proliferation rate until day 2. The same significance has the ratio Day3/Day2 and Day6/Day3.
- %NGFR+ cells before selection indicates the number of transduced cells, genetically modified with the SFCMM-3 vector, described in example 2, coding for Tk molecule and for ΔLNGFR cell surface expression molecule. This value was detected before selection step using a cytofluorimetric analysis.
- MFI is the intensity fluorescence mean and is a parameter of the cytofluorimetric analysis that is related to the quantity of cell molecules expressed on cell surface.
- % of recovery is the ratio between the total cell number collected after selection step and the total cell number of transduced cells before selection step.
- Final percentage of NGFR cells indicates the number of genetically modified cells in the final product.
- N° of final cells is the number of genetically-modified cells collected the day of patient infusion or the day of freezing of the cells.
- The time-points are: day 0, the day of cell stimulation, day 2, the day of transduction, day 6, the day of selection, day 7, the day of beads detachment, day 10, the day of final product recovery.

The experiments can be compared because they were performed starting by a similar number of cells: a mean of 1622x10⁶ cells for the 5 open-system experiments and 932-1000x 10⁶ cells for the closed-system experiments.
**●** The table shows that the experiment conducted with the closed-system, starting from apheresis, is very efficient and the number of genetically-modified cells produced in fact is higher than that produced with the open-system, also starting from apheresis.
● A detailed analysis of the process indicates that the steps in which the closed system shows major advantages are transduction and selection in which the recovery of the cells is very high.
   The purity (final percentage of NGFR positive cells) of the final product is very similar and shows the homogeneity of the final population of genetically-modified cells.
● Only the proliferation rate is lower in closed than open-system, especially between day 3 and 6 and between day 10 and 7. However the development of cell culturing using different bags or different culture media should improve cell proliferation.

These results demonstrate that the present invention provides a method for transduction and selection of genetically-modified cells in a standardized closed system that is safe and efficient.

The present invention tested in the contest of allogeneic bone marrow transplantation clinical protocols can be applied to several gene-therapy clinical studies in which safety and efficiency are necessary.

## Claims

1. A method for transduction of cells with a genetic construct and selection of genetically modified cells, the method comprising performing the transduction and selection in a closed system, wherein the cells are washed, concentrated, transduced and re-suspended using an automated fluid management device without any manual transfer of fluids, and wherein the method comprises immunomagnetic selection of the transduced cells.

2. A method of modifying cells comprising:
(i) optionally thawing said cells;
(ii) optionally stimulating said cells;
(iii) transducing said cells with a genetic construct;
(iv) selecting transduced cells;
(v) optionally expanding and harvesting said transduced cells;
(vi) wherein steps (i) to (v) are performed in a closed system, and wherein step (iv) comprises immunomagnetic selection of transduced cells; and
(vii) wherein the cells are washed, concentrated, transduced and re-suspended using an automated fluid management device without any manual transfer of fluids.

3. A method according to any of the preceding claims wherein the genetic construct is a retroviral vector.

4. A method according to claim 3, wherein the retroviral vector is an MLV derived vector.

5. A method according to claim 3 or 4, wherein the retroviral vector encodes a cell surface marker.

6. A method according to claim 5, wherein the cell surface marker is ΔLNGFR.

7. A method according to any one of the preceding claims wherein the cells are donor T-cells.

8. A method according to any one of the preceding claims wherein the method comprises a transduction step using fibronectin or a variant thereof.

9. A method according to any one of the preceding claims wherein the method comprises a transduction step using the RetroNectin® system.

10. A method according to any one of the preceding claims wherein the method uses a single device.

11. A method according to any one of the preceding claims wherein the method uses two or more devices for cell concentration, cell washing, transduction, medium changing and immunomagnetic selection of transduced cells.

12. A method according to claim 11, wherein the method uses
(i) A first device for cell concentration, cell washing, transduction and medium changing; and
(ii) A second device for immunomagnetic selection of transduced cells.

13. A method according to claim 11 or 12, wherein transfer of cells between the devices is carried out using sealed bags and aseptic connections.

14. A method according to any one of claims 11 to 13, wherein a spinning membrane ensures cell filtration against a counter-flow buffer circulation and is connected to different bags, in a completely closed system that allows for step-by-step user-defmable programming.

15. A method according to claim 13, wherein (i) is performed using a Cytomate™ Cell Processing System or variant thereof.

16. A method according to claim 13, wherein (ii) is performed using an Isolex™ 300 Magnetic Cell Separation System or variant thereof.

## Patentansprüche

1. Verfahren zur Transduktion von Zellen mit einem genetischen Konstrukt und zur Auswahl von genetisch modifizierten Zellen, wobei das Verfahren umfasst, dass man die Transduktion und die Auswahl in einem geschlossenen System durchführt, wobei die Zellen gewaschen, konzentriert, transduziert und re-suspendiert werden, indem man eine automatisierte Fluid-Handhabungsvorrichtung ohne jegliche manuelle Übertragung von Fluiden verwendet, und wobei das Verfahren die immunmagnetische Auswahl der transduzierten Zellen umfasst.

2. Verfahren zum Modifizierten von Zellen, bei dem man:
(i) wahlweise die Zellen auftaut,
(ii) wahlweise die Zellen stimuliert,
(iii) die Zellen mit einem genetischen Konstrukt transduziert,
(iv) die transduzierten Zellen auswählt,
(v) wahlweise die transduzierten Zellen expandiert und erntet,
(vi) wobei die Stufen (i) bis (v) in einem geschlossenen System durchgeführt werden, und wobei die Stufe (iv) die immunmagnetische Auswahl von transduzierten Zellen umfasst, und
(vii) wobei die Zellen gewaschen, konzentriert, transduziert und re-suspendiert werden, indem man eine automatisierte Fluid-Handhabungsvorrichtung ohne jegliche manuelle Übertragung von Fluiden verwendet.

3. Verfahren nach einem der vorangehenden Ansprüche, wobei das genetische Konstrukt ein Retrovirus-Vektor ist.

4. Verfahren nach Anspruch 3, wobei der Retrovirus-Vektor ein von MLV abgeleiteter Vektor ist.

5. Verfahren nach Anspruch 3 oder 4, wobei der Retrovirus-Vektor einen Zelloberflächenmarker kodiert.

6. Verfahren nach Anspruch 5, wobei der Zelloberflächenmarker LNGFR ist.

7. Verfahren nach einem der vorangehenden Ansprüche, wobei die Zellen Donor-T-Zellen sind.

8. Verfahren nach einem der vorangehenden Ansprüche, wobei das Verfahren einen Transduktionsschritt unter Verwendung von Fibronektin oder einer Variante davon umfasst.

9. Verfahren nach einem der vorangehenden Ansprüche, wobei das Verfahren einen Transduktionsschritt unter Verwendung des ReiroNectin®-Systems umfasst.

10. Verfahren nach einem der vorangehenden Ansprüche, wobei man bei dem Verfahren eine einzelne Vorrichtung verwendet.

11. Verfahren nach einem der vorangehenden Ansprüche, wobei man bei dem Verfahren zwei oder mehr Vorrichtungen für die Zellkonzentratian, die Zellwäsche, die Transduktion, den Mediumaustausch und die immunmagnetische Auswahl der transduzierten Zellen verwendet.

12. Verfahren nach Anspruch 11, wobei bei dem Verfahren
(i) eine erste Vorrichtung für die Zellkonzentration, die Zellwäsche, die Transduktion und den Mediumaustausch und
(ii) eine zweite Vorrichtung für die immunmagnetische Auswahl der transduzierten Zellen
verwendet.

13. Verfahren nach Anspruch 11 oder 12, wobei der Transfer von Zellen zwischen den Vorrichtungen unter Verwendung von versiegelten Beuteln und aseptischen Bedingungen durchgeführt wird.

14. Verfahren nach einem der Ansprüche 11 bis 13, wobei eine Spinnmembran die Zellfiltrierung gegen einen Gegenfluss-Pufferkreislauf sicherstellt und mit verschiedenen Beuteln in einem vollständig geschlossenem System, das eine benutzerdefinierte, stufenweise Programmierung ermöglicht, verbunden ist.

15. Verfahren nach Anspruch 13, wobei (i) unter Verwendung eines Zellverarbeitungssystems vom Typ Cytomate^{™} oder einer Variante davon durchgeführt wird.

16. Verfahren nach Anspruch 13, wobei (ii) unter Verwendung eines Systems für die magnetische Zelltrennung vom Typ Isolex^{™} 300 oder einer Variante davon durchgeführt wird.

## Revendications

1. Procédé de transduction de cellules avec une construction génétique et de sélection de cellules modifiées génétiquement, le procédé comprenant la réalisation de la transduction et de la sélection dans un système fermé, les cellules étant lavées, concentrées, transduites et remises en suspension en utilisant un dispositif de gestion automatique de fluide, sans transfert manuel de fluides, et le procédé comprenant une sélection immunomagnétique des cellules transduites.

2. Procédé de modification de cellules, comprenant :
(i) éventuellement, la décongélation desdites cellules ;
(ii) éventuellement, la stimulation desdites cellules ;
(iii) la transduction desdites cellules avec une construction génétique ;
(iv) la sélection de cellules transduites ;
(v) éventuellement, le développement et le recueil desdites cellules transduites ;
(vi) les étapes (i) à (v) étant réalisées dans un système fermé et l'étape (iv) comprenant la sélection immunomagnétique de cellules transduites ; et
(vii) les cellules étant lavées, concentrées, transduites et remises en suspension en utilisant un dispositif automatique de gestion de fluide sans transfert manuel de fluides.

3. Procédé selon l'une quelconque des revendications précédentes, dans lequel la construction génétique est un vecteur rétroviral.

4. Procédé selon la revendication 3, dans lequel le vecteur rétroviral est un vecteur dérivé de MLV.

5. Procédé selon la revendication 3 ou 4, dans lequel le vecteur rétroviral code pour un marqueur de surface cellulaire.

6. Procédé selon la revendication 5, dans lequel le marqueur de surface cellulaire est LNGFR.

7. Procédé selon l'une quelconque des revendications précédentes, dans lequel les cellules sont des lymphocytes T donneurs.

8. Procédé selon l'une quelconque des revendications précédentes, le procédé comprenant une étape de transduction utilisant la fibronectine ou une variante de celle-ci.

9. Procédé selon l'une quelconque des revendications précédentes, le procédé comprenant une étape de transduction utilisant le système RetroNectin®.

10. Procédé selon l'une quelconque des revendications précédentes, ledit procédé utilisant un dispositif unique.

11. Procédé selon l'une quelconque des revendications précédentes, le procédé utilisant deux ou plusieurs dispositifs de concentration cellulaire, de lavage cellulaire, de transduction, de changement de milieu et de sélection immunomagnétique des cellules transduites.

12. Procédé selon la revendication 11, ledit procédé utilisant
(i) un premier dispositif de concentration cellulaire, de lavage cellulaire, de transduction et de changement de milieu ; et
(ii) un deuxième dispositif de sélection immunogène des cellules transduites.

13. Procédé selon la revendication 11 ou 12, dans lequel le transfert de cellules entre les dispositifs est réalisé en utilisant des poches scellées et des raccords aseptiques.

14. Procédé selon l'une quelconque des revendications 11 à 13, dans lequel une membrane de filtrage assure une filtration cellulaire par rapport à une circulation de tampon à contre-courant et est raccordée à différentes poches, dans un système complètement fermé qui permet une programmation pouvant être définie étape par étape par un utilisateur.

15. Procédé selon la revendication 13, dans lequel le point (i) est réalisé à l'aide d'un Système de Traitement de Cellules Cytomate^{™} ou une variante de celui-ci.

16. Procédé selon la revendication 13, dans lequel le point (ii) est réalisé en utilisant un Système de Séparation Magnétique de Cellules Isolex^{™} 300 ou une variante de celui-ci.
